# EUROPÄISCHE PATENTSCHRIFT

(11) **EP 0 207 882 B1**
(45) Veröffentlichungstag und Bekanntmachung des Hinweises auf die Patenterteilung: **02.01.1997**
(21) Anmeldenummer: 86730096.4
(22) Anmeldetag: 20.06.1986
(51) Int. Cl.: A61K 31/48

(54) **Verwendung von Tergurid zur Herstellung eines Arzneimittels zur Behandlung geriatrischer Beschwerden**
Use of terguride for the manufacture of a medicament for the treatment of geriatric infirmities
Utilisation de la terguride pour la fabrication d'un médicament pour le traitement de maladies gériatriques

(30) Priorität: 24.06.1985 DE 3522894
(43) Veröffentlichungstag der Anmeldung: 07.01.1987
(73) Patentinhaber: SCHERING AKTIENGESELLSCHAFT, 13342 Berlin (DE)
(72) Erfinder: Aufdembrinke, Bernd, Dr., D-1000 Berlin 28 (DE); Dorow, Rainer, D-1000 Berlin 31 (DE); Horowski, Reinhard, Dr., D-1000 Berlin 28 (DE); Suchy, Irmgard, Dr., D-1000 Berlin 19 (DE); Schröder, Gertrud, Dr., D-1000 Berlin 42 (DE); Wachtel, Helmut, Dr., D-1000 Berlin 19 (DE); Kehr, Wolfgang, Dr., D-1000 Berlin 19 (DE); Stock, Günter, Dr., D-1000 Berlin 37 (DE)

(56) Entgegenhaltungen:
- EP-A- 0 021 206
- EP-A- 0 056 358
- EP-A- 0 118 848
- GB-A- 2 116 548
- ADVANCES IN NEUROLOGY, Band 45, 8th Int. Symp. Parkinson's Disease, 9-12. Juni, 1985, S. 573-576, Raven Press, New York, US; T. BRÜCKE et al.: "Terguride: partial dopamine agonist in the treatment of Parkinson's disease"
- ADVANCES IN NEUROLOGY, Band 45, 8th Int. Symp. Parkinson's Disease, 9-12. Juni 1985, Seiten 577-582, Raven Press, New York, US; I. SUCHY et al.: "Evaluation of terguride in patients with Parkinson's disease"
- CLIN. NEUROPHARMACOL., Band 7, Suppl. 1, 1984, Seiten 950-951; Raven Press, New York, US G.U. CORSINI et al.: "Treatment of Parkinson's disease with a dopamine partial agonist"
- J. NEUROL., Band 232, Suppl. 1985, Seite 163, Ref. Nr. 11.21.02; M. ESTEGUY et al.: "Treatment of Parkinson's disease with transdihydrolisuride"
- LIFE SCIENCE, Band 32, 1983, Seiten 421-432, Pergamon Press, US; H. WACHTEL et al.: "Dual action on central dopamine function of transdiydrolisuride, A9,10- dihydrogenated analogue of the Ercodopamine agonist lisuride"
- THE MERCK MANUAL, Band 14, 1982, S. 1359-1362, 1462-1471; Ed. R. BERKOW, Merck & Co., Rahway, US
- Römpps Chemie-Lexikon, 8. Auflage, 1981, S.1453
- Allgemeine und Spezielle Pharmakologie und Toxikologie, S. 569-572
- The Merck Manual, 14. Auflage, 1982, Seiten 1360 und 2226
- Dorland's Illustrated Medical Dictionary, 28. Auflage, 1981, Seite547
- Pschyrembel Klinisches Wörterbuch, 1977, S. 1113

## Beschreibung

Die Erfindung betrifft die Verwendung von Tergurid oder dessen physiologisch verträglichen Salzen zur Herstellung eines Arzneimittels zur Behandlung geriatrischer Beschwerden mit Ausnahme des Morbus Parkinson.

Als physiologisch verträgliche Salze kommen Salze von Tergurid mit anorganischen und organischen Säuren in Frage. Zur Salzbildung geeignet sind zum Beispiel Salzsäure, Phosphorsäure, Schwefelsäure, Methansulfonsäure, Glucoheptansäure, Bernsteinsäure, Weinsäure, Maleinsäure usw. Ein bevorzugtes Salz ist das Tergurid-dihydrogenphosphat.

Tergurid (3-(6-Methylergolin-8α-yl)-1.1-diethylharnstoff) selbst und dessen nidations- und laktationshemmende sowie dessen antipsychotische Wirkung bei oraler Anwendung am Tier und am Menschen aufgrund seiner partialagonistischen Wirkung an Dopaminrezeptoren sind bekannt (DE PS 22 38 540, DE OS 31 29 714).

Weiterhin sind auch verschiedene Mutterkornalkaloide, wie z.B. Bromocriptin oder Lisurid, in der Humanmedizin zur Hochdrucktherapie verwendet worden (Stumpe, K.O., Kolloch,R., Higuchi, M.K., Kruck, F., Vetter, H.: Hyperprolactinemia and antihypertensive effect of bromocryptine in essential hypertension, Lancet 2:211, 1977).

Des weiteren ist bekannt, daß der für motorische, aber auch psychische, cognitive und endokrine Funktionen wichtige Neurotransmitter Dopamin mit zunehmendem Alter sowohl am Versuchstier als auch beim Menschen in seiner Konzentration im Gehirn und mit zunehmendem Alter in seiner Wirksamkeit abfällt (A. Carlsson and Winblad, B., J. Neural Transmission 38, 271-276, 1976; Severson, J.A. and Finch, C.E., Brain Research 192, 147-162, 1980). Als Extremform des Dopaminmangels im motorischen System ist seit langem der Morbus Parkinson bekannt, für dessen Therapie sich Dopaminagonisten wie L-Dopa und Bromocriptin bewährt haben.

Diese Substanzen haben jedoch den Nachteil, daß sie derart stark auf alle Dopaminrezeptoren wirken, daß sie als Nebenwirkung auch Übelkeit, Erbrechen, orthostatische Dysregulation und Benommenheit hervorrufen.

Die Anwendung anderer Mutterkornalkaloide mit schwach dopaminerger Wirksamkeit - die erst bei einer Langzeitbehandlung, z.B. als Prolaktinsenkung feststellbar ist - wie das Dihydroergotoxin, das zur Therapie für cognitive und Vigilanzstörungen sowie anderer Störungen der Hirnfunktion im Alter eingesetzt werden kann, ist ebenfalls bekannt (R.J. McDonald, Pharmacopsychiatry 12, 407-422, 1979).

Dieser schwach wirksame Dopaminagonist hat jedoch den Nachteil, daß seine ausgeprägte α-adrenolytische Wirkung die Anwendung höherer Dosierungen, wie sie nötig wären, um rasch eine stärkere therapeutische Wirkung zu erreichen, verbietet. Eine akut höhere Dosierung von Dihydroergotoxin ist nicht möglich, da dann neben der dopaminerg vermittelten Blutdrucksenkung starke orthostatische Reaktionen auftreten.

Die Aufgabe der vorliegenden Erfindung war es, ein Mittel zur Behandlung von geriatrischen Beschwerden bereitzustellen, welches zur Behandlung der durch die eingeschränkte Dopaminfunktion im Alter mitverursachten Einschränkungen von Vigilanz, Stimmung und psychomotorischen Funktionen geeignet ist.

Es wurde überraschenderweise gefunden, daß Tergurid bei unterschiedlichen Ausgangsbedingungen die dopaminerge Neurotransmission in unterschiedlicher Weise beeinflußt. So läßt sich eine Dopaminunterfunktion - z.B. durch chronische Reserpingabe in der Ratte oder beim Morbus Parkinson - durch Tergurid ausgleichen, während intakte Dopaminsysteme durch vergleichbare Dosierungen nicht beeinflußt werden. Daher sind die Nebenwirkungen geringer und Symptome wie sie durch Stimulation des Dopaminrezeptors mit klassischen Dopaminagonisten beoabachtet werden, sind nicht zu erwarten. Es ist sogar möglich Systeme mit Dopaminüberfunktion - z.B. durch Amphetamin- oder Lisuridbehandlung an der Ratte oder beim Morbus Huntington oder Schizophrenie - durch Tergurid zu hemmen, ohne daß andere dopaminerge Systeme mit normaler Funktion beeinflußt werden. Es war daher nicht vorhersehbar, daß Tergurid als Partialdopaminagonist zur Behandlung von Dopaminunterfunktion und auch zur Behandlung von Dopaminüberfunktion verwendet werden kann.

Somit ist die Anwendung von Tergurid bei Cerebralinsuffizienz im Alter, Unterfunktion dopaminerger Systeme, zur Regulierung der Motorik, Stimmung und Vigilanz möglich, ohne daß hierdurch - wie im Falle der klassischen Dopaminagonisten - schwer erträgliche subjektive Nebenwirkungen ausgelöst werden. Die zusätzliche Möglichkeit auch Dopaminüberfunktion zu normalisieren, ist auch nützlich, wenn die Überfunktion durch dopaminerge Arzneimittel ausgelöst wurde, z.B. bei Morbus Parkinson.

Klinisch zeigte sich in kontrollierten Studien bei alten Patienten mit Morbus Parkinson durch eine Behandlung mit Tergurid nicht nur eine Verbesserung der Motorik, sondern zugleich auch der Stimmung und Vigilanz. Bei Patienten mit den klinischen Zeichen einer Demenz ließen sich im Elektroencephalogramm (EEG)
Frequenzveränderungen nachweisen, die den mit dem Altern verbundenen EEG-Frequenzänderungen entgegenliefen.

Bei alten Probanden , die gleichzeitig an Hochdruck litten, wurden die allgemeine Leistungsfähigkeit und das Wohlbefinden durch eine Reduktion des erhöhten systolischen und diastolischen Blutdrucks nicht beeinträchtigt. Eine schonende, langsam sich entwickelnde antihypertensive Wirkung ohne gleichzeitig auftretende Änderungen der Herzfrequenz trägt darüberhinaus dazu bei, die durch Hypertonie bedingten Gefäßkomplikationen zu reduzieren.

In allen Fällen und im Gegensatz zur Therapie mit Dopaminagonisten waren die subjektive und objektive Verträglichkeit von Tergurid sehr gut. Im Gegensatz zur sonstigen Therapie ließen sich die positiven Aspekte der Behandlung bereits in den ersten Tagen feststellen.

Eine sofortige dopaminerge Stimulation durch Tergurid ließ sich auch durch die prolaktinsenkende Wirkung dieser Behandlung zeigen. Ein Effekt, der nach der Behandlung mit Dihydroergotoxin erst nach Wochen oder Monaten eintritt und dann mit dem klinischen Therapieerfolg bei Cerebralinsuffizienz im Alter korreliert ist. Orthostatische Dysregulationen werden in den verabreichten Dosierungen nicht beobachtet.

Die klinischen Ergebnisse sind in der nachfolgenden Tabelle dargestellt.

**TABELLE**

| | Hydergin (Dihydroergotoxin) | Tergurid | Bromocriptin Lisurid |
|---|---|---|---|
| antihypertensiver Effekt | ++ | ++ | + |
| prolaktinsenkender Effekt | (+) nach Wochen | ++ | ++ |
| Obelkeit, Erbrechen | - | - | ++ |
| Orthostase | + | (+) | + |
| Vigilanzverbesserung | nach Wochen | nach Tagen | durch Nebenwirkungen nicht feststellbar (sowohl Aktivierung wie Dysphorie) |
| Stimmungsverbesserung | nach Wochen | nach Tagen | |
| - nicht beobachtet bei normalen Dosisbereichen (+) sehr selten beobachtet (bei höheren Dosen) + vorhanden (aber meist bei höheren Dosen) ++ ausgeprägt bei normalen Dosisbereichen | | | |

Die Daten in der Tabelle zeigen, daß Tergurid im Vergleich zu Bromocriptin besser verträglich ist. Im Vergleich zum Hydergin ist Tergurid stärker wirksam, wobei die Wirkung auch noch rascher eintritt. Die Anwendung von Tergurid bereitet im Vergleich zur Anwendung der beiden Standardsubstanzen weniger Orthostase-Probleme.

Die erfindungsgemäße Verwendung von Tergurid im Vergleich zu den vorhandenen Therapiemöglichkeiten mit starken Dopaminagonisten und ihren starken Nebenwirkungen einerseits und dem schwachen Dopaminagonisten Dihydroergotoxin mit starker α-adrenolytischer Komponente und zu geringer und später Wirkung (Loew et al., Aging 23, 227-239, 1983) bedeutet bei einer Behandlung der Cerebralinsuffizienz im Alter und anderer Folgen eines funktionellen Dopaminmangels, mit Tergurid somit eine erhebliche Verbesserung.

In der medizinischen Praxis können Arzneimittel auf Basis von Tergurid per oral oder patenteral wie subcutan, intramuskulär und intravenös appliziert werden. Bevorzugt ist die per orale Applikation. Die tägliche Dosis beträgt 0,1 - 5,0 mg, bevorzugt 0,25 - 1,0 mg.

Die Herstellung der Arzneimittelspezialitäten erfolgt in an sich bekannter Weise, indem das Tergurid mit den in der Pharmazie gebräuchlichen Trägersubstanzen, Verdünnungsmitteln, Geschmackskorrigentien usw. verarbeitet wird. Für Injektionen kommen insbesondere wässrige, aber auch ölige Lösungen sowie Suspensionen in Frage. Zur Herstellung intramuskulärer Depotformen können die Wirkstoffe nach gängigen Methoden in fetten Ölen suspendiert oder gelöst werden.

Tergurid kann auch in für die Erhaltung von gleichmäßigen Plasmaspiegeln üblichen galenischen Zubereitungen verwendet werden wie z.B. für die Dauerinfusion mit gebräuchlichen Pumpen in wässrigen oder anderen geeigneten Lösungsmitteln in einer Dosis von 0,01 - 10 mg/kg. Den oralen und parentalen Formulierungen können andere Stoffe wie beispielsweise Dopaminagonisten zugesetzt werden, um die Wirkung zu erhöhen oder Nebenwirkungen zu verringern .

Die erfindungsgemäßen Arzneimittel sind insbesondere in Form von Tabletten, Kapseln, Dragees, Pillen, Suspensionen und Lösungen für die orale Applikation geeignet. Geeignet sind aber auch orale Retardformen, die in üblicher Weise, z.B. durch Zugabe von hydrierten Fetten und Verarbeitung mit Harzbildnern und Lacken, erhalten werden. Tropfen für die orale Applikation können durch wässrige Lösungen oder Suspensionen des Wirkstoffes in Ölen unter Zugabe von Geschmackskorrigenten und/oder Lösungsvermittlern hergestellt werden.

## Patentansprüche

1. Verwendung von Tergurid oder dessen physiologisch verträglichen Salzen zur Herstellung eines Arzneimittels zur Behandlung geriatrischer Beschwerden mit Ausnahme des Morbus Parkinson.

2. Verwendung gemäß Anspruch 1, dadurch gekennzeichnet, daß cognitive und psychische Störungen behandelt werden.

3. Verwendung gemäß Anspruch 1 dadurch gekennzeichnet, daß Einschränkungen der Stimmung, Vigilanz, der Psychomotorik und Cerebralinsuffizienz behandelt werden.

4. Verwendung gemäß Anspruch 2, dadurch gekennzeichnet, daß Demenz behandelt wird.

## Claims

1. Use of terguride or the physiologically tolerable salts thereof for the preparation of a medicament for the treatment of geriatric complaints, with the exception of Parkinson's disease.

2. Use according to claim 1, characterised in that cognitive and psychic disorders are treated.

3. Use according to claim 1, characterised in that limitations of mood, attentiveness, of psychomotor functions and cerebral insufficiency are treated.

4. Use according to claim 2, characterised in that dementia is treated.

## Revendications

1. Utilisation de terguride ou de ses sels physiologiquement tolérés pour la préparation d'un médicament pour le traitement de troubles gériatriques, à l'exception de la maladie de Parkinson.

2. Utilisation selon la revendication 1, caractérisée en ce qu'il s'agit de troubles cognitifs et psychiques.

3. Utilisation selon la revendication 1, caractérisée en ce que l'on traite les troubles d'humeur, de diminution de vigilance, des fonctions psychomotrices et d'insuffisance cérébrale.

4. Utilisation selon la revendication 2, caractérisée en ce que l'on traite la démence.
